# EUROPEAN PATENT SPECIFICATION

(11) **EP 3 013 327 B1**
(45) Date of publication and mention of the grant of the patent: **14.05.2025**
(21) Application number: 14817510.2
(22) Date of filing: 30.06.2014
(51) Int. Cl.: A61K 9/48, A61K 31/4985, A61K 31/18, A61P 13/08

(54) **PHARMACEUTICAL CAPSULE COMPOSITE FORMULATION COMPRISING TADALAFIL AND TAMSULOSIN**
PHARMAZEUTISCHE KAPSELVERBUNDSTOFFFORMULIERUNG MIT TADALAFIL UND TAMSULOSIN
PRÉPARATION COMPOSITE POUR GÉLULE PHARMACEUTIQUE COMPRENANT DU TADALAFIL ET DE LA TAMSULOSINE

(30) Priority: 28.06.2013 KR 20130076073
(43) Date of publication of application: 04.05.2016
(73) Proprietor: Hanmi Pharm. Co., Ltd., Gyeonggi-do 445-910 (KR)
(72) Inventor: PARK, Caleb Hyungmin, Goyang-si Gyeonggi-do 412-818 (KR); KIM, Yong Il, Suwon-si, Gyeonggi-do 16325 (KR); PARK, Jae Hyun, Suwon-si, Gyeonggi-do 16703 (KR); WOO, Jong Soo, Suwon-si Gyeonggi-do 440-710 (KR); YOON, Young-su, Changwon-si Gyeongsangnam-do 642-820 (KR)
(74) Representative: Marks & Clerk LLP
(86) International application number: PCT/KR2014/005813
(87) International publication number: WO 2014/209087

(56) References cited:
- WO-A1-2006/108519
- WO-A1-2013/055177
- KR-A- 20070 072 982
- KR-A- 20080 007 252
- KR-A- 20130 039 797
- US-A1- 2005 008 690
- US-A1- 2010 221 321
- ELI LILLY COMPANY: "FDA- HIGHLIGHTS OF PRESCRIBING INFORMATION: CIALIS (tadalafil) tablets for oral administration", 1 January 2008 (2008-01-01), XP055872454, Retrieved from the Internet <URL:https://www.accessdata.fda.gov/drugsatfda_docs/label/2008/021368s011lbl.pdf> [retrieved on 20211213]
- ANONYMOUS: "Flomax® (tamsulosin hydrochloride) Capsules, 0.4 mg Prescribing Information", DRUGS@FDA INSTRUCTIONS: REGULATORY INFORMATION, 5 October 2005 (2005-10-05), pages 1 - 20, XP055664689, Retrieved from the Internet <URL:https://www.accessdata.fda.gov/drugsatfda_docs/label/2005/020579s016lbl.pdf> [retrieved on 20200204]

## Description

### FIELD OF THE INVENTION

The present invention relates to a capsule composite formulation containing tadalafil and tamsulosin as defined in claim 1. The capsule composite formulation is suitable for preventing or treating erectile dysfunction and benign prostatic hyperplasia. The invention also relates to a method of manufacturing the capsule composite formulation as defined in claim 7.

### BACKGROUND OF THE INVENTION

Erectile dysfunction and benign prostatic hyperplasia are diseases common to males in their 50's or older. Erectile dysfunction is a sexual dysfunction characterized by the inability to develop or maintain an erection of the penis during sexual performance due to various causes including cardiovascular diseases, diabetes, hormonal deficiency, etc. Benign prostatic hyperplasia is an increase in size of the prostate which causes dysuresia, with the concomitant or consequent onset of complications such as urinary infection, urolithiasis, hematuria, renal failure, etc.

Tadalafil is a substance belonging to phosphodiesterase 5 (PDE 5) inhibitors such as sildenafil and vardenafil. Tadalafil has a half life at least 3 times longer than sildenafil and vardenafil. Tadalafil was originally developed by Icos Corporation. Eli Lilly and Company currently offers Cialis^{®}, a treatment for erectile dysfunction containing tadalafil, and Adcirca^{®}, a treatment for pulmonary arterial hypertension, on the market. Cialis^{®} was approved in 2011 by the FDA as a treatment for benign prostatic hyperplasia.

Tamsulosin is an α1a blocker effective in the treatment of symptoms of benign prostatic hyperplasia, chronic prostatitis, and chronic abdominal pain. In addition, tamsulosin is also effective in the treatment of urolithiasis via the skeletal muscle relaxation mechanism by blocking α1a. Tamsulosin was first developed by Yamanouchi Pharmaceutical Co., Ltd. in 1996, and various products containing tamsulosin hydrochloride are known (Korean Patent Laid-open Publication No. 2006-105976, etc.).

Erectile dysfunction and benign prostatic hyperplasia may occur alone and independent of each other. However, erectile dysfunction and benign prostatic hyperplasia are likely to occur in the same patient, and, according to a study, 8.5 patients out of 10 erectile dysfunction patients in Korea also had prostate gland diseases. Accordingly, there is a need for the development of a therapeutic method to treat the two diseases simultaneously with excellent stability and efficacy.

In particular, although the action mechanism of tadalafil differs from that of tamsulosin, they are both effective in the treatment of benign prostatic hyperplasia. Therefore, a more excellent therapeutic effect may be obtained by administering both tadalafil and tamsulosin simultaneously or at intervals as a combined therapy, and also adverse effects due to long term administration may be alleviated by reducing the dosage of each individual drug. However, the combined therapy, which requires administration of at least two drugs as individual units may deteriorate drug compliance, and thus may cause much inconvenience to patients who are under continuous medication. In addition, the combined therapy also causes much inconvenience to patients who continuously manage their social activities by requiring them to carry with them and administer several individual drug units.

Accordingly, there is an urgent need for the development of a composite formulation (also called as a combination drug) containing at least two active ingredients necessitating a combined therapy. However, the development of a composite formulation containing at least two active ingredients raises problems as follows. First, the composite formulation requires that the different active ingredients to be used therein be easily and freely combined but unexpected difficulties may occur due to various problems caused by the pharmacokinetic and pharmaceutical characteristics of the drugs. Second, the amount of a composition containing the active ingredients and a pharmaceutically acceptable excipient should be in the range suitable as a medicine. Therefore, when the amount of active ingredients to be combined is excessive or too little it may be difficult to prepare them into a composition with an appropriate mass. Third, in manufacturing a combination drug, the dissolution rate and stability of the composite formulation may be deteriorated by the interaction between the different active ingredients of the composite formulation, thus making it difficult to develop a fixed composite formulation for administration in a physicochemically stable form. The composite formulation may be manufactured in the form of a double-layered or a triple-layered tablet to separate each active ingredient into each separate layer. However, the above method will require a special manufacturing facility such as a tableting machine for preparing double-layered or triple-layered tablets, and also interactions may occur among the ingredients in neighboring regions of the tablets. In the contemporary art, it has not been possible to establish a perfect separation between ingredients.

WO 2006/108519 A1 discloses a method for treating the symptoms of benign prostatic hyperplasia.

WO 2013/055177 A1 discloses a hard capsule composite formulation comprising one or more tablets encapsulated in the capsule, wherein the tablet or the tablets as a whole have a shape conforming to the internal space of the capsule.

EPO non-patent literature reference number XP055872455 is a press release regarding a phase III clinical trial evaluating administration of Cialis in parallel with tamsulosin to men with signs and symptoms suggestive of benign prostatic hyperplasia.

W. B. White and T. Moon, The Journal of Clinical Hypertension, 2005, 7, 212-217 discusses treatment of benign prostatic hyperplasia in hypertensive men.

EPO non-patent literature reference number XP055872454 discloses prescribing information for Cialis.

EPO non-patent literature reference number XP055664689 discloses prescribing information for Flomax (tamsulosin hydrochloride).

Accordingly, there has been a need for the development of a novel composite formulation with excellent medication convenience and stability, while being capable of providing the effects of preventing and treating erectile dysfunction and benign prostatic hyperplasia.

### SUMMARY OF THE INVENTION

Accordingly, an object of the present invention is to provide a composite formulation with excellent medication convenience, dissolution rate and stability, while being capable of providing the effects of preventing and treating both erectile dysfunction and benign prostatic hyperplasia.

In order to accomplish the above object(s), the present invention provides a capsule composite formulation comprising: an independent part of tadalafil containing tadalafil or a pharmaceutically acceptable salt thereof; and an independent part of tamsulosin containing tamsulosin or a pharmaceutically acceptable salt thereof, in a separate state. The independent part of tadalafil is in the form of a tablet, and the independent part of tamsulosin is in the form of granules. The independent part of tamsulosin is coated with a coating material selected from the group consisting of povidone, propylene glycol, polyvinyl acetate, methacrylate-ethyl acrylate copolymer, triacetin, and a mixture thereof. The capsule composite formulation is filled into a hard capsule that has an internal capacity of 0.47mL, and the coating material is used in the amount of 0.1 to 20 wt% relative to the total weight of the independent part of tamsulosin.

In order to accomplish another object, the present invention also provides a method of manufacturing the above capsule composite formulation as defined in claim 7. The method comprises: a) mixing tadalafil or a pharmaceutically acceptable salt thereof with a pharmaceutically acceptable additive, and tableting the granules thus obtained into a tablet; b) mixing tamsulosin or a pharmaceutically acceptable salt thereof with a pharmaceutically acceptable additive, and granulating the mixture; and c) filling into a hard capsule the tadalafil tablet prepared in step a), and the tamsulosin granules prepared in step b), in a separate state. Step b) includes coating the granules obtained from the granulation process with a coating material selected from the group consisting of povidone, propylene glycol, polyvinyl acetate, methacrylate-ethyl acrylate copolymer, triacetin, and a mixture thereof. The capsule composite formulation is filled into a hard capsule that has an internal capacity of 0.47mL, and the coating material is used in the amount of 0.1 to 20 wt% relative to the total weight of the independent part of tamsulosin.

The capsule composite formulation of the present invention may be prepared by efficiently filling a pharmaceutical composition into the limited internal capacity of a capsule. Therefore, the capsule composite formulation of the present invention has advantages in that it can provide high-dose active ingredients into a small sized capsule, thereby having high productivity and providing patients with improved convenience in taking the medicine. Additionally, the capsule composite formulation of the present invention has an excellent dissolution rate because the pharmaceutically active ingredients within the capsule are separated, thus having a low impact on the dissolution rate between the pharmaceutically active ingredients. Furthermore, the capsule composite formulation of the present invention has minimal reactivity between active ingredients, thus providing excellent product stability over time and is capable of maximizing the therapeutic effect of the pharmaceutically active ingredients.

### BRIEF DESCRIPTION OF THE DRAWINGS

The above and other objects and features of the present invention will become apparent from the following description of the invention, when taken in conjunction with the accompanying drawings:
FIG. 1 is a diagram of a capsule composite formulation prepared according to an exemplary embodiment of the present invention;
FIG. 2 and FIG. 3 respectively show the results of a dissolution test for tadalafil and tamsulosin conducted according to Test Example 1;
FIG. 4 and FIG. 5 respectively show the individual impurities and total impurities of tadalafil according to Test Example 2; and
FIG. 6 and FIG. 7 respectively show the individual impurities and total impurities of tamsulosin according to Test Example 2.

### DETAILED DESCRIPTION OF THE INVENTION

The present invention is described in further detail below.

The term, "a composite formulation" used herein, refers to a formulation which includes at least two kinds of drugs or active ingredients within a single dosage unit such as a table or capsule.

The capsule composite formulation of the present invention is as defined in claim 1 and comprises i) an independent part of tadalafil containing tadalafil or a pharmaceutically acceptable salt thereof; and ii) an independent part of tamsulosin containing tamsulosin or a pharmaceutically acceptable salt thereof, in a separate state.

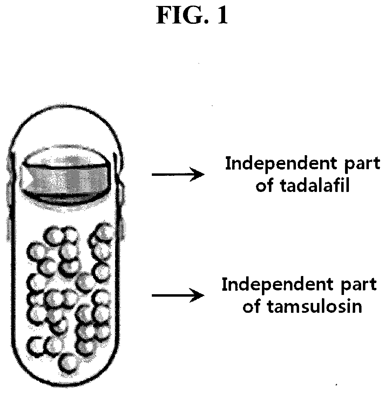

FIG. 1 is a diagram illustrating the capsule composite formulation according to an exemplary embodiment of the present invention, in which the independent part of tadalafil and the independent part of tamsulosin respectively form a separate independent layer within the hard capsule to be filled. More specifically, in an exemplary embodiment, the capsule composite formulation may comprise i) an independent tadalafil layer containing tadalafil or a pharmaceutically acceptable salt thereof; and ii) an independent tamsulosin layer containing tamsulosin or a pharmaceutically acceptable salt thereof, in a separate state.

The capsule composite formulation of the present invention has a therapeutic effect of preventing or treating erectile dysfunction and benign prostatic hyperplasia.

The independent part of tadalafil and the independent part of tamsulosin may contain water in the range of 5% or less, respectively.

The pharmaceutically acceptable salt of tamsulosin may be, for example, tamsulosin hydrochloride.

The capsule composite formulation comprises a tadalafil tablet and a tamsulosin granule filled into a hard capsule. A diagram of a capsule composite formulation according to an embodiment of the present invention which comprises a tadalafil tablet and a tamsulosin granule filled into a hard capsule is shown in Fig. 1.

Tadalafil or a pharmaceutically acceptable salt thereof may be contained in the range of from 3 to 7 wt% relative to the total weight of the independent part of tadalafil. Preferably, tadalafil or a pharmaceutically acceptable salt thereof may be administered to an adult at about 5 mg daily.

Tamsulosin or a pharmaceutically acceptable salt thereof may be contained in the range of from 0.1 to 0.2 wt% relative to the total weight of the independent part of tamsulosin. Tamsulosin or a pharmaceutically acceptable salt thereof may be administered to an adult, for example, at about 0.2 mg or 0.4 mg daily.

In the capsule composite formulation of the present invention, the independent part of tadalafil and the independent part of tamsulosin may each independently contain a pharmaceutically acceptable additive, respectively, for example, a diluent, a disintegrating agent, a binder, a stabilizing agent, a lubricant, a coloring agent, or a mixture thereof.

Examples of the diluent may include microcrystalline cellulose, lactose, Ludipress, mannitol, monocalcium phosphate, starch, low-substituted hydroxypropylcellulose, and a mixture thereof. The diluent may be used in the amount of from about 1 to 95 wt% relative to the total weight of each independent part, and preferably from about 5 to 95 wt%.

Examples of the disintegrating agent may include crosspovidone, sodium starch glycolate, sodium crosscarmellose, low-substituted hydroxypropylcellulose, starch, alginic acid or a sodium salt thereof, and a mixture thereof, which can serve for stable disintegration of active ingredients. The disintegrating agent may be used in the amount of from about 0.1 to 30 wt% relative to the total weight of each independent part, and preferably from about 2 to 15 wt%.

Examples of the binder may include hydroxypropylcellulose, hydroxypropyl methylcellulose, hypromellose, polyvinyl acetate, polyvinyl pyrrolidone, copovidone, macrogol, sodium lauryl sulfate, light anhydrous silicic acid, synthetic aluminum silicate, a silicate derivative such as calcium silicate or magnesium metasilicate aluminate, phosphate such as calcium hydrogen phosphate, carbonate such as calcium carbonate, pregelatinized starch, gums such as acasia gum, gelatin, cellulose derivatives such as ethyl cellulose, and a mixture thereof. The binder may be used in the amount of from about 0.1 to 30 wt% relative to the total weight of each independent part, and preferably from about 2 to 20 wt%.

Examples of the lubricant may include metal stearates such as stearic acid, calcium stearate, or magnesium stearate; talc; colloidal silica; sucrose fatty acid ester; hydrogenated vegetable oil; high melting point wax; glyceryl fatty acid esters; glycerol dibehenate; and a mixture thereof. The lubricant may be used in the amount of from about 0.3 to 5 wt% relative to the total weight of each independent part, and preferably from about 0.5 to 3 wt%.

In the present invention, the independent part of tadalafil and the independent part of tamsulosin may be each coated with a pharmaceutically acceptable coating material. The coating material to be used may include any polymer conventionally used in the related art. For example, the independent part of tadalafil may be coated by the commercial Opadry^{®} manufactured by Colorcon Ltd. or the like as the coating material. The coating material for the independent part of tamsulosin is selected from the group consisting of polyvinyl acetate, povidone, methacrylate-ethyl acrylate copolymer, triacetin, propylene glycol, and a mixture thereof.

Preferably, the amount of the coating material should be maintained at the minimal level for the preparation of optimal-sized formulations and their efficient manufacture. For example, the coating material may be used in the amount of from about 0.1 to 20 wt% relative to the total weight of each independent part, and preferably from about 2 to 10 wt%. In the invention, the coating material on the independent part of the tamsulosin is used in the amount of 0.1 to 20 wt% relative to the total weight of the independent part of tamsulosin.

The capsules to be used in manufacturing the capsule composite formulation of the present invention are not particularly limited, but any conventional hard capsules used in medicinal products may be used. For example, any hard capsule containing hypromellose, pullulan, gelatin, polyvinyl alcohol, or a mixture thereof may be used.

Capsules used in medicinal products have a varying amount of an internal capacity depending on the size number, for example, size No. 0 has about 0.68 mL, size No. 1 about 0.47 mL, size No. 2 about 0.37 mL, size No. 3 about 0.27 mL, size No. 4 about 0.20 mL, etc.

The capsule composite formulation of the present invention employs tadalafil, a PDE 5 inhibitor, as the first active ingredient, thereby having therapeutic effects of preventing and treating erectile dysfunction and benign prostatic hyperplasia, whereas it employs tamsulosin, an α1a blocker, as the second active ingredient, thereby having continuous therapeutic effects for preventing and treating dysuresia diseases, such as benign prostatic hyperplasia, chronic prostatitis, chronic abdominal pain, urolithiasis, etc.

Suitable routes for administration of the capsule composite formulation of the present invention may include oral, buccal, and sublingual routes.

In the capsule composite formulation of the present invention, a pharmaceutical composition is efficiently filled into a capsule having a limited internal capacity, and thus it is possible to fill high-dose active ingredients into a small-sized capsule. Accordingly, the capsule composite formulation of the present invention can be manufactured with high productivity, and it also provides patients with improved convenience in taking medicines. Additionally, the two active ingredients, tadalafil and tamsulosin, are included within a hard capsule in a separate state, and thus the two ingredients can be completely separated. Therefore, the two active ingredients have little mutual impact on their dissolution rate, thus enabling an overall excellent dissolution rate.

Furthermore, the minimized reactivity between the active ingredients contributes to product stability over time thereby maximizing the therapeutic effect, and also an existing analysis method for the evaluation of stability over time of a single formulation can be used, without necessities for developing additional methods.

The capsule composite formulation of the present invention may be manufactured by a method including: a) mixing tadalafil or a pharmaceutically acceptable salt thereof with a pharmaceutically acceptable additive, and tableting the mixture; b) mixing tamsulosin or a pharmaceutically acceptable salt thereof with a pharmaceutically acceptable additive, and granulating the mixture; and c) filling the tadalafil tablet prepared in step a), and the tamsulosin granules prepared in step b) into a hard capsule, in a separate state.

In step a), tablets may be manufactured by tableting the granules obtained by granulation. More specifically, tablets may be manufactured using a tableting machine according to a conventional method. Preferably, the tablets thus manufactured may have a suitable hardness, for example, an average hardness in the range of from 1 to 30 kp after tableting.

Step a) includes tableting, and thus, tadalafil is filled into a capsule in the form of a tablet. For example, the capsule composite formulation may be manufactured by performing tableting in step a), performing granulation in step b) and filling a tadalafil tablet and a tamsulosin granule, in a separate state, into a hard capsule.

Additionally, the method of manufacturing a capsule composite formulation may further include coating the tablet, obtained by the tableting in step a), with a pharmaceutically acceptable coating material. For example, the capsule composite formulation may be manufactured by performing coating after tableting of tadalafil in step a), performing coating after granulation of tamsulosin in step b), and filling a coated tadalafil tablet and a coated tamsulosin granule, in a separate state, into a hard capsule. Examples of the coating material suitable for tadalafil and tamsulosin are the same as described above.

The present invention is further described and illustrated in examples provided below, which are, however, not intended to limit the scope of the present invention.

### Example 1: A capsule composite formulation I

### <Independent part of tadalafil>

| | |
|---|---|
| tadalafil | 5.0 mg |
| mannitol | 54.8 mg |
| hydroxypropylcellulose | 3.3 mg |
| sodium lauryl sulfate | 0.3 mg |
| microcrystalline cellulose | 16.1 mg |
| sodium starch glycolate | 4.5 mg |
| Magnesium stearate | 1.1mg |
| Opadry^{®} yellow | 2.5 mg |
| purified water | (38.0 mg) |

### <Independent part of tamsulosin>

| | |
|---|---|
| tamsulosin hydrochloride | 0.2 mg |
| polyvinyl acetate dispersion | 22.84 mg |
| microcrystalline cellulose | 123.5 mg |
| hypromellose | 5.5 mg |
| povidone | 0.36 mg |
| propylene glycol | 0.27 mg |
| methacrylate-ethyl acrylate copolymer | 2.05 mg |
| triacetin | 0.36 mg |
| sucrose stearate | 0.2 mg |
| purified water | (100.6 mg) |

The ingredients in powder form, corresponding to the independent part of tadalafil, were mixed, and the mixture was tableted by a circular punch having a diameter of 5.5 mm. The resulting tadalafil tablets were coated with a coating solution, i.e., a solution of Opadry^{®} yellow (Colorcon Ltd.) in purified water.

Additionally, the ingredients corresponding to the independent part of tamsulosin were mixed in powder form, and the mixture was prepared into granules. The resulting tamsulosin granules were coated with an inner coating solution, i.e., a solution of povidone, propylene glycol and polyvinyl acetate in water, and then coated further with an external coating solution, i.e., a solution of methacrylate-ethyl acrylate copolymer and triacetin in water.

The tadalafil tablets and the tamsulosin granules thus coated were filled into hard capsules No. 1 having hypromellose as a capsule material, and manufactured into a capsule composite formulation containing 5 mg of tadalafil and 0.2 mg of tamsulosin hydrochloride.

### Example 2: A capsule composite formulation II

A capsule composite formulation containing 5 mg of tadalafil and 0.2 mg of tamsulosin hydrochloride was manufactured in the same manner as in Example 1, except that the hard capsule used had pullulan as a capsule material.

### Example 3: A capsule composite formulation III

A capsule composite formulation containing 5 mg of tadalafil and 0.2 mg of tamsulosin hydrochloride was manufactured in the same manner as in Example 1, except that the hard capsule used had gelatin as a capsule material.

### Comparative Example 1: A simple mixed tablet composite formulation

| | |
|---|---|
| tadalafil | 5.0 mg |
| tamsulosin hydrochloride | 0.2 mg |
| mannitol | 54.8 mg |
| hydroxypropylcellulose | 3.3 mg |
| sodium lauryl sulfate | 0.3 mg |
| microcrystalline cellulose | 16.1 mg |
| sodium starch glycolate | 4.5 mg |
| Magnesium stearate | 1.1mg |
| Opadry^{®} yellow | 2.5 mg |
| purified water | (38.0 mg) |

A mixture of the above ingredients was subjected to wet granulation using a binder, i.e., a solution of hydroxypropylcellulose and sodium lauryl sulfate in water, and then sieved with a 30 mesh sieve and dried.

The dried resultant was added with mannitol, microcrystalline cellulose, sodium starch glycolate, and magnesium stearate, and then tableted by a tableting machine.

The tablet thus obtained which contains tadalafil and tamsulosin was then coated with a coating solution, i.e., a solution of Opadry^{®} yellow in purified water. As a result, a simple mixed tablet composite formulation containing 5 mg of tadalafil and 0.2 mg of tamsulosin hydrochloride was obtained.

### Comparative Example 2: A double-layered tablet composite formulation

### <Tadalafil-containing layer>

| | |
|---|---|
| tadalafil | 5.0 mg |
| mannitol | 54.8 mg |
| hydroxypropylcellulose | 3.3 mg |
| sodium lauryl sulfate | 0.3 mg |
| microcrystalline cellulose | 16.1 mg |
| sodium starch glycolate | 4.5 mg |
| Magnesium stearate | 1.1mg |
| Opadry^{®} yellow | 2.5 mg |
| purified water | (38.0 mg) |

### <Tamsulosin containing layer>

| | |
|---|---|
| tamsulosin hydrochloride | 0.2 mg |
| polyvinyl acetate dispersion | 22.84 mg |
| microcrystalline cellulose | 123.5 mg |
| hypromellose | 5.5 mg |
| povidone | 0.36 mg |
| propylene glycol | 0.27 mg |
| methacrylate-ethyl acrylate copolymer | 2.05 mg |
| triacetin | 0.36 mg |
| sucrose stearate | 0.2 mg |
| purified water | (100.6 mg) |

First, in order to prepare a tadalafil-containing layer, tadalafil was subjected to wet granulation using a binder, i.e., a solution of hydroxypropylcellulose and sodium lauryl sulfate in water, and then sieved with a 30 mesh sieve and dried. The dried resultant was added with mannitol, microcrystalline cellulose, sodium starch glycolate, and magnesium stearate, and then tableted by a tableting machine.

Additionally, the ingredients for the tamsulosin-containing layer were mixed and then tableted along with the tadalafil tablet prepared in advance to manufacture a double-layered tablet.

The double-layered tablet thus obtained was then coated with a coating solution, i.e., a solution of Opadry^{®} yellow in purified water. As a result, a double-layered tablet composite formulation containing 5 mg of tadalafil and 0.2 mg of tamsulosin hydrochloride was obtained.

### Test Example 1: Evaluation of dissolution

The tadalafil and tamsulosin hydrochloride composite formulations prepared in Examples 1 - 3 and Comparative Examples 1 and 2 were evaluated for their dissolution according to the conditions described below.

### <Tadalafil dissolution conditions>

A dissolution test was performed according to the Paddle method of the U. S. Pharmacopeia (USP) dissolution test using 1000 mL of 0.5% sodium lauryl sulfate (SLS). Samples for the dissolution test were collected at the initial stage, and after 5 min, 10 min, 15 min, 30 min, 45 min and 60 min, respectively, and the tadalafil dissolution rate was measured via liquid chromatography under the conditions described below.
- column: a column in which a stainless steel tube having an inner diameter of about 4.6 mm and a length of about 5 cm is filled with an octadecylsilyl (ODS)-silica gel for liquid chromatography having a particle size of 3.5 µm (Zorbax SB-C8, Agilent Zorbax)
- detector: UV spectrophotometer (measured at 225nm of wavelength)
- flow rate: 2.0 mL/min
- input volume: 50 µL
- column temperature: 40°C
- mobile phase: water/methanol (50:50, v/v)
- dissolution medium: 1000 mL of 0.5% sodium lauryl sulfate (SLS)

### <Tamsulosin dissolution conditions>

A dissolution test was performed according to the Paddle method of the USP dissolution test with a sinker at 100 rpm using 500 mL of the second fluid for disintegration test, i.e., pH 6.8 buffer. Samples for the dissolution test were collected in the amount of 10 mL at the initial stage, and after 15 min, 30 min, 60 min, 90 min, 120 min, 180 min, 300 min, 360 min, and 480 min, respectively. The tamsulosin dissolution rate of the samples thus collected was measured via liquid chromatography under the conditions described below.
- column: a column in which a stainless steel tube having an inner diameter of about 4.6 mm and a length of about 15 cm is filled with an octadecylsilyl (ODS)-silica gel for liquid chromatography having a particle size of 5 µm (Zorbax SB-C8, Agilent Zorbax)
- detector: UV spectrophotometer (measured at 225nm of wavelength)
- flow rate: adjusted to retain tamsulosin for about 6 min
- input volume: 500 µL
- column temperature : 40°C
- mobile phase: 8.7 mL of perchloric acid and 3.0 g of sodium hydroxide were dissolved in 1900 mL of water. The mixture was adjusted to pH 2.0 with sodium hydroxide, and then added with water to 2000 mL. 1400 mL of the resulting solution was added with 600 mL of acetonitrile to obtain a mobile phase.
- dissolution medium: 500 mL of the second fluid for disintegration test (17 g of KH₂PO₄ and 16.75 g of Na₂HPO₄ were dissolved in 10 L of purified water to prepare pH 6.8 buffer).

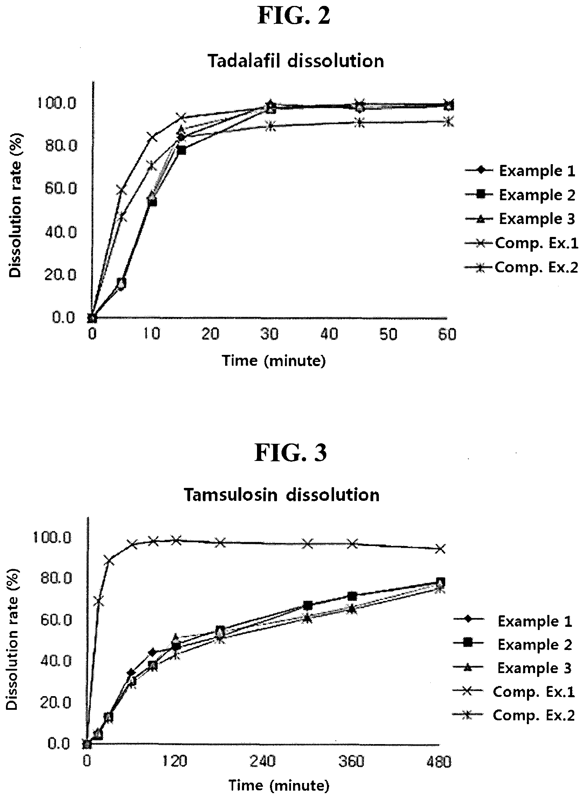

The result of tadalafil dissolution is shown in Table 1 and FIG. 2, and the result of tamsulosin dissolution is shown in Table 2 and FIG. 3.

**[Table 1]**

| | Tadalafil dissolution rate | | | | | | |
|---|---|---|---|---|---|---|---|
| | 0 min | 5 min | 10 min | 15 min | 30 min | 45 min | 60 min |
| Ex.1 | 0.0% | 14.7% | 55.9% | 84.6% | 99.8% | 98.0% | 99.2% |
| Ex.2 | 0.0% | 16.9% | 54.1% | 78.5% | 97.8% | 99.1% | 99.5% |
| Ex.3 | 0.0% | 16.2% | 57.6% | 88.0% | 98.1% | 98.9% | 99.3% |
| Comp. Ex.1 | 0.0% | 59.5% | 84.7% | 93.5% | 98.6% | 100.1% | 99.8% |
| Comp. Ex.2 | 0.0% | 47.3% | 70.9% | 84.4% | 89.6% | 91.3% | 92.2% |

As shown in Table 1 and FIG. 2, the capsule composite formulations prepared in Examples 1 to 3 and the simple mixed tablet composite formulations prepared in Comparative Example 1 revealed excellent tadalafil dissolution rates. However, in the case of the double-layered tablet composite formulation prepared in Comparative Example 2, the tadalafil dissolution rate was decreased by about 5% or more because part of tadalafil was in contact with a sustained release agent needed for tamsulosin.

**[Table 2]**

| | Tamsulosin dissolution rate | | | | | | | | | |
|---|---|---|---|---|---|---|---|---|---|---|
| | 0 min | 15 min | 30 min | 60 min | 90 min | 120 min | 180 min | 300 min | 360 min | 480 min |
| Ex.1 | 0.0% | 5.8% | 13.5% | 34.6% | 44.8% | 47.0% | 52.5% | 67.1% | 72.1% | 78.8% |
| Ex.2 | 0.0% | 4.9% | 13.2% | 30.9% | 38.4% | 48.6% | 55.8% | 67.7% | 72.0% | 79.2% |
| Ex.3 | 0.0% | 5.8% | 13.4% | 31.4% | 38.8% | 51.9% | 54.7% | 62.3% | 66.7% | 78.0% |
| Comp. Ex.1 | 0.0% | 69.3% | 89.3% | 96.7% | 98.3% | 99.1% | 98.1% | 97.2% | 97.1% | 95.3% |
| Comp. Ex.2 | 0.0% | 4.8% | 12.4% | 29.1% | 37.4% | 43.5% | 51.4% | 61.3% | 65.7% | 75.7% |

As shown in Table 2 and FIG. 3, the capsule composite formulations prepared in Examples 1 to 3 and the double-layered composite formulation prepared in Comparative Example 2 revealed excellent tamsulosin dissolution rates. However, in the case of the simple mixed tablet composite formulation prepared in Comparative Example 1, tamsulosin was dissolved quickly due to the absence of a sustained release agent, but the formulation was not suitable for therapeutic treatment because of the short half-life of tamsulosin.

In light of the dissolution test results above, the capsule composite formulations of the present invention can provide excellent dissolution of both tadalafil and tamsulosin for lack of interaction between them because the two active ingredients, tadalafil and tamsulosin, are filled into the capsule in a separate state. On the contrary, in the case of the simple mixed tablet composite formulation and the double-layered composite formulation, the dissolution of tadalafil or tamsulosin was poor due to the interaction between the ingredients or the problems in the manufacture of the composite formulations.

### Test Example 2: Impurity test

Composite formulations containing tadalafil and tamsulosin hydrochloride prepared in Examples 1 - 3 and Comparative Examples 1 and 2 were tested for their impurities according to the conditions described below.

### <Accelerated conditions>

accelerated storage condition: at 40 °C with 75% of relative humidity test timing: initial stage, after 1 month, 3 months, and 6 months

### <Tadalafil impurity test conditions>

Tests were performed according to the USP impurity test. The formulation corresponding to 100 mg of tadalafil was added to a 100 mL flask, which was then filled to half with a mobile phase and shaken for 15 minutes. The resultant was dissolved by ultrasonic vibration for about 2 minutes, mixed with a mobile phase to adjust its volume, and then filtered. The resultant in the amount of 5 mL was mixed again with 20 mL of the mobile phase to prepare a sample liquid having a final concentration of 0.25 mg/mL, and tadalafil impurities were measured by liquid chromatography according to the conditions described below.
- column: a column in which a stainless steel tube having an inner diameter of about 4.6 mm and a length of about 15 cm is filled with an octadecylsilyl (ODS)-silica gel for liquid chromatography having a particle size of 3.5 µm (Zorbax SB-C8, Agilent Zorbax)
- detector: UV spectrophotometer (measured at 285nm of wavelength)
- flow rate: 1.0 mL/min
- input volume: 10 µL
- column temperature : 40°C
- mobile phase: 0.1% trifluoroacetic acid-acetonitrile/purified water (35:65, v/v)
- time for analysis: 30min

### <Tamsulosin impurity test conditions>

Tests were performed according to the USP impurity test. The formulation corresponding to 4 mg of tamsulosin was added to a 25 mL flask, which was then diluted with a mobile phase. Exactly 10 mL of the resulting solution was taken and added into a 25 mL flask, to obtain a diluted sample. Tamsulosin impurities were measured by liquid chromatography under the conditions described below.

### before main peak

- column: a column in which a stainless steel tube having an inner diameter of about 4.6 mm and a length of about 15 cm is filled with an octadecylsilyl (ODS)-silica gel for liquid chromatography having a particle size of 3.5 µm (Zorbax SB-C8, Agilent Zorbax)
- detector: UV spectrophotometer (measured at 225nm of wavelength)
- flow rate: adjusted to retain tamsulosin for about 8 min
- input volume: 100 µL
- column temperature: 40°C
- mobile phase: acetonitrile/buffer solution = 3:7 (the buffer solution was prepared by dissolving 8.7 mL of 70% perchloric acid and about 3.0 g of NaOH in 1900 mL of purified water, adjusting the pH of the mixture to pH 2.0 with 1N NaOH, and then diluting it with 2000 mL of purified water)
- time for analysis: 25 min

### after main peak

- column: a column in which a stainless steel tube having an inner diameter of about 4.6 mm and a length of about 15 cm is filled with an octadecylsilyl (ODS)-silica gel for liquid chromatography having a particle size of 5 µm (Zorbax SB-C8, Agilent Zorbax)
- detector: UV spectrophotometer (measured at 225nm of wavelength)
- flow rate: 1.0 mL/min
- input volume: 100 µL
- column temperature: 40°C
- mobile phase: acetonitrile/buffer solution = 1:1 (the buffer solution was prepared by dissolving 8.7 mL of 70% perchloric acid and about 3.0 g of NaOH in 1900 mL of purified water, adjusting the pH of the mixture to pH 2.0 with 1N NaOH, and then diluting it with 2000 mL of purified water)
- time for analysis: 15 min

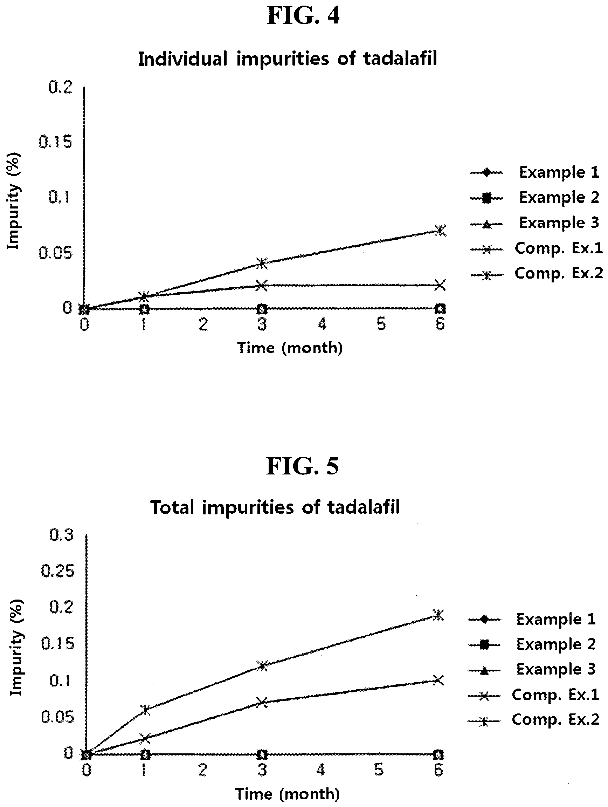

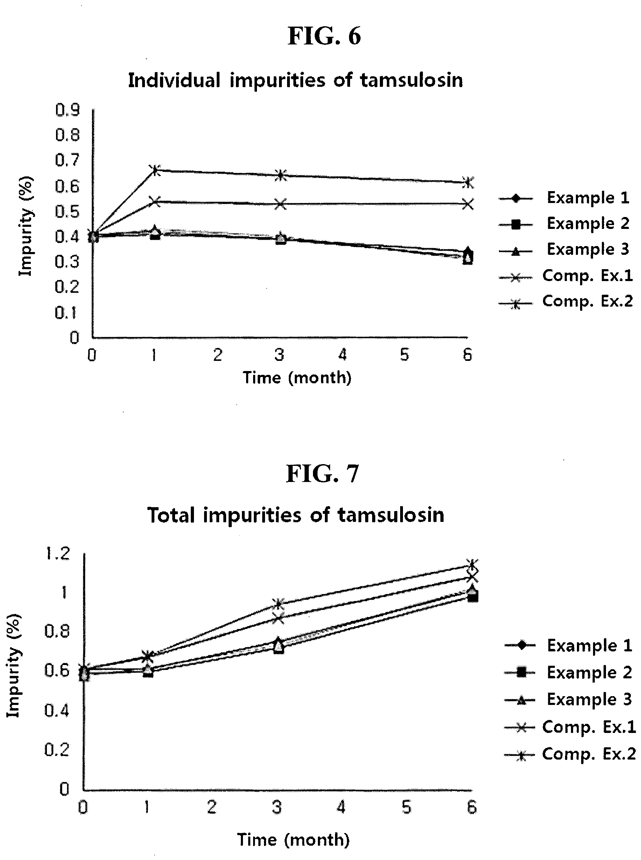

The amount of tadalafil impurities are shown in Tables 3 and 4 and FIGS. 4 and 5. The amount of tamsulosin impurities are shown in Tables 5 and 6 and FIGS. 6 and 7.

**[Table 3]**

| | Individual impurities of tadalafil | | | |
|---|---|---|---|---|
| | Initial stage | Accelerated 1 month | Accelerated 3 months | Accelerated 6 months |
| Ex.1 | 0.00% | 0.00% | 0.00% | 0.00% |
| Ex.2 | 0.00% | 0.00% | 0.00% | 0.00% |
| Ex.3 | 0.00% | 0.00% | 0.00% | 0.00% |
| Comp. Ex.1 | 0.00% | 0.01% | 0.02% | 0.02% |
| Comp. Ex.2 | 0.00% | 0.01% | 0.04% | 0.07% |

**[Table 4]**

| | Total impurities of tadalafil | | | |
|---|---|---|---|---|
| | Initial stage | Accelerated 1 month | Accelerated 3 months | Accelerated 6 months |
| Ex.1 | 0.00% | 0.00% | 0.00% | 0.00% |
| Ex.2 | 0.00% | 0.00% | 0.00% | 0.00% |
| Ex.3 | 0.00% | 0.00% | 0.00% | 0.00% |
| Comp. Ex.1 | 0.00% | 0.02% | 0.07% | 0.10% |
| Comp. Ex.2 | 0.00% | 0.06% | 0.12% | 0.19% |

As shown in Tables 3 and 4 and FIGS. 4 and 5, the capsule composite formulations prepared in Examples 1 to 3 exhibited excellent tadalafil impurity levels and satisfied the USP standards for individual impurities and total impurities, i.e., 0.2% or below and 0.3% or below, respectively. However, in the case of the simple mixed tablet composite formulations prepared in Comparative Example 1, and the double-layered tablet composite formulations prepared in Comparative Example 2, the amount of impurities increased because part of tadalafil was in contact with tamsulosin or its excipients.

**[Table 5]**

| | Individual impurities of tamsulosin | | | |
|---|---|---|---|---|
| | Initial stage | Accelerated 1 month | Accelerated 3 months | Accelerated 6 months |
| Ex.1 | 0.41% | 0.42% | 0.39% | 0.34% |
| Ex.2 | 0.40% | 0.41% | 0.39% | 0.32% |
| Ex.3 | 0.40% | 0.43% | 0.40% | 0.31% |
| Comp. Ex.1 | 0.41% | 0.54% | 0.53% | 0.53% |
| Comp. Ex.2 | 0.41% | 0.66% | 0.64% | 0.61% |

**[Table 6]**

| | Total impurities of tamsulosin | | | |
|---|---|---|---|---|
| | Initial stage | Accelerated 1 month | Accelerated 3 months | Accelerated 6 months |
| Ex.1 | 0.61% | 0.61% | 0.75% | 1.01% |
| Ex.2 | 0.59% | 0.60% | 0.72% | 0.98% |
| Ex.3 | 0.58% | 0.62% | 0.73% | 1.02% |
| Comp. Ex.1 | 0.61% | 0.67% | 0.87% | 1.08% |
| Comp. Ex.2 | 0.61% | 0.68% | 0.94% | 1.14% |

As shown in Tables 5 and 6 and FIGS. 6 and 7, the capsule composite formulations prepared in Examples 1 to 3 exhibited excellent tamsulosin impurity levels and satisfied the USP standards for individual impurities and total impurities, i.e., 0.9% or below and 1.1% or below, respectively. However, in the case of the simple mixed tablet composite formulations prepared in Comparative Example 1, and the double-layered tablet composite formulations prepared in Comparative Example 2, the amount of impurities increased because part of tamsulosin was in contact with tadalafil or its excipients.

While the invention has been described with respect to the above specific embodiments, it should be recognized that various modifications and changes may be made to the invention by those skilled in the art which also fall within the scope of the invention as defined by the claims.

## Claims

1. A capsule composite formulation comprising:
i) an independent part of tadalafil containing tadalafil or a pharmaceutically acceptable salt thereof; and
ii) an independent part of tamsulosin containing tamsulosin or a pharmaceutically acceptable salt thereof,
in a separate state,
wherein the independent part of tadalafil is in the form of a tablet, and the independent part of tamsulosin is in the form of granules,
wherein the independent part of tamsulosin is coated with a coating material selected from the group consisting of povidone, propylene glycol, polyvinyl acetate, methacrylate-ethyl acrylate copolymer, triacetin, and a mixture thereof, wherein the capsule composite formulation is filled into a hard capsule that has an internal capacity of 0.47mL, and wherein the coating material is used in the amount of 0.1 to 20 wt% relative to the total weight of the independent part of tamsulosin.

2. The capsule composite formulation of claim **1,** wherein the independent part of tadalafil and the independent part of tamsulosin further comprise a pharmaceutically acceptable additive selected from the group consisting of a diluent, a disintegrating agent, a binder, a stabilizing agent, a lubricant, a coloring agent, and a mixture thereof.

3. The capsule composite formulation of claim 1, wherein the independent part of tadalafil and the independent part of tamsulosin respectively contain 5% or less of water content.

4. The capsule composite formulation of claim 1, wherein the hard capsule comprises hypromellose, pullulan, gelatin, polyvinyl alcohol or a mixture thereof as a capsule material.

5. The capsule composite formulation of claim 1, wherein the pharmaceutically acceptable salt of tamsulosin is tamsulosin hydrochloride.

6. A capsule composite formulation as defined in any one of claims 1 to 5 for use in the prevention or treatment of erectile dysfunction and benign prostatic hyperplasia.

7. A method of manufacturing the capsule composite formulation of claim 1, comprising:
a) mixing tadalafil or a pharmaceutically acceptable salt thereof with a pharmaceutically acceptable additive, and tableting the granules thus obtained into a tablet;
b) mixing tamsulosin or a pharmaceutically acceptable salt thereof with a pharmaceutically acceptable additive, and granulating the mixture; and
c) filling into a hard capsule the tadalafil tablet prepared in step a), and the tamsulosin granules prepared in step b), in a separate state;
wherein step b) includes coating the granules obtained from the granulation process with a coating material selected from the group consisting of povidone, propylene glycol, polyvinyl acetate, methacrylate-ethyl acrylate copolymer, triacetin, and a mixture thereof, wherein the capsule composite formulation is filled into a hard capsule that has an internal capacity of 0.47mL, and wherein the coating material is used in the amount of 0.1 to 20 wt% relative to the total weight of the independent part of tamsulosin.

8. The method of manufacturing the capsule composite formulation of claim 7, wherein step a) includes coating of the tablet obtained from the tableting process with a pharmaceutically acceptable coating material.

## Patentansprüche

1. Kapselverbundstoffformulierung, Folgendes umfassend:
i) einen unabhängigen Anteil Tadalafil, enthaltend Tadalafil oder ein pharmazeutisch annehmbares Salz davon; und
ii) einen unabhängigen Anteil Tamsulosin, enthaltend Tamsulosin oder ein pharmazeutisch annehmbares Salz davon,
in einem getrennten Zustand,
wobei der unabhängige Anteil Tadalafil in Form einer Tablette vorliegt und der unabhängige Anteil Tamsulosin in Form von Granulat vorliegt,
wobei der unabhängige Anteil Tamsulosin mit einem Beschichtungsmaterial beschichtet ist, ausgewählt aus der Gruppe, bestehend aus Povidon, Propylenglycol, Polyvinylacetat, Methacrylat-Ethylacrylat-Copolymer, Triacetin und einer Mischung davon,
wobei die Kapselverbundstoffformulierung in eine Hartkapsel gefüllt wird, die ein Innenvolumen von 0,47 ml aufweist, und wobei das Beschichtungsmaterial in einer Menge von 0,1 bis 20 Gew.-% in Bezug auf das Gesamtgewicht des unabhängigen Anteils Tamsulosin verwendet wird.

2. Kapselverbundstoffformulierung nach Anspruch 1, wobei der unabhängige Anteil Tadalafil und der unabhängige Anteil Tamsulosin ferner einen pharmazeutisch annehmbaren Zusatzstoff umfassen, ausgewählt aus der Gruppe bestehend aus einem Verdünnungsmittel, einem Zerfallmittel, einem Bindemittel, einem Stabilisator, einem Schmiermittel, einem Färbemittel und einer Mischung davon.

3. Kapselverbundstoffformulierung nach Anspruch 1, wobei der unabhängige Anteil Tadalafil und der unabhängige Anteil Tamsulosin jeweils einen Wassergehalt von 5 % oder weniger enthalten.

4. Kapselverbundstoffformulierung nach Anspruch 1, wobei die Hartkapsel Hypromellose, Pullulan, Gelatine, Polyvinylalkohol oder eine Mischung davon als Kapselmaterial umfasst.

5. Kapselverbundstoffformulierung nach Anspruch 1, wobei das pharmazeutisch annehmbare Salz von Tamsulosin Tamsulosin-Hydrochlorid ist.

6. Kapselverbundstoffformulierung wie in einem der Ansprüche 1 bis 5 definiert, zur Verwendung bei der Vorbeugung oder Behandlung von erektiler Dysfunktion und gutartiger Prostatahyperplasie.

7. Verfahren zur Herstellung der Kapselverbundstoffformulierung nach Anspruch 1, Folgendes umfassend:
a) Mischen von Tadalafil oder eines pharmazeutisch annehmbaren Salzes davon mit einem pharmazeutisch annehmbaren Zusatzstoff und Tablettieren des so erzielten Granulats zu einer Tablette;
b) Mischen von Tamsulosin oder eines pharmazeutisch annehmbaren Salzes davon mit einem pharmazeutisch annehmbaren Zusatzstoff und Granulieren der Mischung; und
c) Einfüllen der in Schritt a) hergestellten Tadalafil-Tablette und des in Schritt b) hergestellten Tamsulosin-Granulats, in einem getrennten Zusand, in eine Hartkapsel;
wobei Schritt b) das Beschichten der in dem Granulierungsprozess erhaltenen Granulate mit einem Beschichtungsmaterial umfasst, das ausgewählt ist aus der Gruppe, bestehend aus Povidon, Propylenglycol, Polyvinylacetat, Methacrylat-Ethylacrylat-Copolymer, Triacetin und einer Mischung davon, wobei die Kapselverbundstoffformulierung in eine Hartkapsel gefüllt wird, die ein Innenvolumen von 0,47 ml aufweist, und wobei das Beschichtungsmaterial in einer Menge von 0,1 bis 20 Gew.-% in Bezug auf das Gesamtgewicht des unabhängigen Anteils Tamsulosin verwendet wird.

8. Verfahren zur Herstellung der Kapselverbundstoffformulierung nach Anspruch 7, wobei Schritt a) das Beschichten der durch das Tablettieren erzielten Tablette mit einem pharmazeutisch annehmbaren Beschichtungsmaterial einschließt.

## Revendications

1. Formulation composite pour gélule comprenant :
i) une partie indépendante de tadalafil contenant du tadalafil ou un sel pharmaceutiquement acceptable de celui-ci ; et
ii) une partie indépendante de tamsulosine contenant de la tamsulosine ou un sel pharmaceutiquement acceptable de celle-ci,
dans un état séparé,
dans laquelle la partie indépendante de tadalafil se présente sous la forme d'un comprimé et la partie indépendante de tamsulosine se présente sous la forme de granulés,
dans laquelle la partie indépendante de tamsulosine est enrobée d'un matériau d'enrobage sélectionné parmi le groupe constitué de la povidone, du propylène glycol, de l'acétate de polyvinyle, du copolymère de méthacrylate-acrylate d'éthyle, de la triacétine et d'un mélange de ceux-ci,
dans laquelle la formulation composite pour gélule est versée dans une gélule dure qui présente une capacité interne de 0,47 ml et dans laquelle le matériau d'enrobage est utilisé en une quantité de 0,1 à 20 % en poids par rapport au poids total de la partie indépendante de tamsulosine.

2. Formulation composite pour gélule selon la revendication 1, dans laquelle la partie indépendante de tadalafil et la partie indépendante de tamsulosine comprennent en outre un additif pharmaceutiquement acceptable sélectionné parmi le groupe constitué d'un diluant, d'un désintégrant, d'un liant, d'un stabilisant, d'un lubrifiant, d'un colorant et d'un mélange de ceux-ci.

3. Formulation composite pour gélule selon la revendication 1, dans laquelle la partie indépendante de tadalafil et la partie indépendante de tamsulosine contiennent respectivement une teneur en eau de 5 % ou moins.

4. Formulation composite pour gélule selon la revendication 1, dans laquelle la gélule dure comprend de l'hypromellose, du pullulane, de la gélatine, de l'alcool polyvinylique ou un mélange de ceux-ci en tant que matériau de gélule.

5. Formulation composite pour gélule selon la revendication 1, dans laquelle le sel pharmaceutiquement acceptable de tamsulosine est le chlorhydrate de tamsulosine.

6. Formulation composite pour gélule tel que défini dans l'une quelconque des revendications 1 à 5 pour utilisation dans la prévention ou le traitement d'une dysfonction érectile et d'une hyperplasie bénigne de la prostate.

7. Procédé de fabrication de la formulation composite pour gélule selon la revendication 1, comprenant :
a) le mélange du tadalafil ou d'un sel pharmaceutiquement acceptable de celui-ci avec un additif pharmaceutiquement acceptable et la transformation des granulés ainsi obtenus en un comprimé ;
b) le mélange de la tamsulosine ou d'un sel pharmaceutiquement acceptable de celle-ci avec un additif pharmaceutiquement acceptable et la transformation du mélange en granulés ; et
c) le versement dans une gélule dure du comprimé de tadalafil préparé à l'étape a) et des granulés de tamsulosine préparés à l'étape b), dans un état séparé ;
dans lequel l'étape b) inclut l'enrobage des granulés obtenus à partir du processus de granulation avec un matériau d'enrobage sélectionné parmi le groupe constitué de la povidone, du propylène glycol, de l'acétate de polyvinyle, du copolymère de méthacrylate-acrylate d'éthyle, de la triacétine et d'un mélange de ceux-ci, dans lequel la formulation composite pour gélule est versée dans une gélule dure qui présente une capacité interne de 0,47 ml et dans lequel le matériau d'enrobage est utilisé en une quantité de 0,1 à 20 % en poids par rapport au poids total de la partie indépendante de tamsulosine.

8. Procédé de fabrication de la formulation composite pour gélule selon la revendication 7, dans lequel l'étape a) inclut l'enrobage du comprimé obtenu à partir du processus de fabrication de comprimés avec un matériau d'enrobage pharmaceutiquement acceptable.
